(19) 

Europäisches
Patentamt
European
Patent Office
Office européen
des brevets

(11) **EP 4 512 888 A1**

(12) **EUROPEAN PATENT APPLICATION**
published in accordance with Art. 153(4) EPC

(43) Date of publication:
**26.02.2025 Bulletin 2025/09**

(21) Application number: **23791980.8**

(22) Date of filing: **26.01.2023**

(51) International Patent Classification (IPC):
*C12N 1/12* (2006.01)    *C12P 1/00* (2006.01)
*C12R 1/89* (2006.01)

(52) Cooperative Patent Classification (CPC):
**C12N 1/12; C12P 1/00;** C12R 2001/89

(86) International application number:
**PCT/KR2023/001183**

(87) International publication number:
**WO 2023/204396 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.04.2022 KR 20220047721**

(71) Applicant: **CJ CheilJedang Corporation**
**Seoul 04560 (KR)**

(72) Inventors:
• **SHIN, Won Sub**
 **Seoul 04560 (KR)**

• **CHOI, Jung-Woon**
 **Seoul 04560 (KR)**
• **JANG, Sunghoon**
 **Seoul 04560 (KR)**
• **GWAK, Jun Seok**
 **Seoul 04560 (KR)**
• **KANG, Hae-Won**
 **Seoul 04560 (KR)**
• **RYU, Ae Jin**
 **Seoul 04560 (KR)**
• **KIM, Ji Young**
 **Seoul 04560 (KR)**

(74) Representative: **Reddie & Grose LLP**
**The White Chapel Building**
**10 Whitechapel High Street**
**London E1 8QS (GB)**

(54) **HIGH-PROTEIN MICROALGAL BIOMASS HAVING EXCELLENT PEPSIN DIGESTIBILITY, CULTURE METHOD AND USE THEREOF**

(57) The present invention relates to high-protein microalgal biomass having excellent pepsin digestibility, a culture method and use thereof. A composition disclosed in the present invention has a high protein content, but also contains omega-3 fatty acids, which are useful fatty acids similar to those in fish meal, and thus can directly replace fish meal when mixing animal feed.

【FIG. 4】

Total amino acid vs Pepsin digestibility
$R^2 = 0.9527$

EP 4 512 888 A1

**Description**

[TECHNICAL FIELD]

Cross-reference to related application(s)

The present application claims the benefit of the priority based on Korean Patent Application No. 10-2022-0047721 filed on April 18, 2022, and the entire contents disclosed in the documents of the corresponding Korean patent application are incorporated as a part of the present description.

The present invention relates to high-protein microalgal biomass having excellent pepsin digestibility, its culture method and uses thereof.

[BACKGROUND ART]

Fishmeal is a protein source that is widely used in fish feed as well as feed for various livestock species. However, development of a new protein source that can replace fishmeal is required as environmental pollution problems such as regulations on catches, heavy metals and microplastics have recently been raised.

In addition, there is a recent trend to reduce the FIFO (Fish in/Fish out) ratio raised in the field of fish feed, and in addition, as the amount of catch varies depending on government policy or climate, there is a growing demand for an improved protein source in terms of sustainability.

Until now, various new protein sources have been proposed as substitutes for fishmeal, but there was a problem in that they could not replace omega-3 fatty acids, and the like which are effective substances contained in fishmeal. Furthermore, a green algae-based whole cell type protein presented so fat has a problem in that it is practically difficult to comprise a large proportion in the feed mixing ratio because the digestibility is greatly reduced. In this regard, Korean Patent No. 1564829 discloses a formulated feed composition for fish farming containing microalgae byproducts.

Accordingly, as a result of research to solve these problems, the present inventors have developed a new protein source comprising omega-3 fatty acids which are useful fatty acids produced through fermentation of Thraustochytrid-based microalgae. Furthermore, they have found a composition having pepsin digestibility and have proposed a new sustainable and environmentally friendly protein source.

[DISCLOSURE]

[TECHNICAL PROBLEM]

One embodiment of the present application provides biomass derived from the *Schizochytrium* sp. microalgae having excellent pepsin digestibility comprising 46% or more of total amino acids based on the biomass dry weight.

Another embodiment of the present application provides a feed composition comprising the biomass derived from the *Schizochytrium* sp. microalgae.

Other embodiment of the present application provides a food composition comprising the biomass derived from the *Schizochytrium* sp. microalgae.

Other embodiment of the present application provides a method for culturing *Schizochytrium* sp. microalgae, comprising; culturing *Schizochytrium* sp. microalgae under a condition in which a concentration of a residual carbon source in a culture solution is maintained at 6% or less compared to the total culture solution.

[TECHNICAL SOLUTION]

Each description and embodiment disclosed in the present application can also be applied to each other description and embodiment. In other words, all combinations of various elements disclosed in the present application fall within the scope of the present application. In addition, the scope of the present application is not be construed as being limited by specific description described below. In addition, those skilled in the art will recognize or confirm many equivalents to specific aspects of the present application described in the present application using only an ordinary experiment. Moreover, such equivalents are intended to be comprised in the present application.

One embodiment of the present application provides *Schizochytrium* sp. microalgae having excellent pepsin digestibility comprising total amino acids of 46 % by weight or more compared to the biomass dry weight.

The term used in the present description, "Thraustochytrid" means microalgae of the Thraustochytriales order. In addition, the term used in the present description, "*Schizochytrium* sp." is one of species names belonging to the Thraustochytriaceae family of the Thraustochytriales order, and may be interchangeably used with "Schizochytrium sp. (genus *Schizochytrium*)." Moreover, the term "microalgae" is small algae that cannot be seen with naked eyes and can only be seen through a microscope, and means a living organism that floats and lives freely in water. There are various types in the microalgae, and it includes strains that cannot photosynthesize and grow only as heterotrophs.

The *Schizochytrium* sp. microalgae may be *Schizochytrium* sp. CD01-5004 strain deposited with accession number KCTC14345BP, but not limited thereto.

The term used in the present description, "biomass" means an energy source of an organism such as a plant, an animal, a microorganism which can be used as chemical energy, that is, bioenergy, and also means the weight or energy amount of a specific organism that exists within a unit time and space ecologically. In addition, the biomass may comprise a compound secreted by cells, but not limited thereto, and contain not only extracellular substances but also cells and/or intracellular contents. In the present application, the biomass may be *Schizochytrium* sp. microalgae itself, a culture product thereof, a dry matter thereof, a lysate thereof or a product produced by culturing or fermenting the microalgae, or may be a concentrate or dry matter of the biomass, but not limited thereto.

Herein, the "Culture product" of the *Schizochytrium* sp. microalgae refers to a product produced by culturing the microalgae, and specifically, it may be a culture solution comprising microalgae or culture filtrate in which microalgae is removed in the culture solution, but not limited thereto. The "Dry matter" of the *Schizochytrium* sp. microalgae culture is one in which moisture is removed in the microalgae culture, and for example, it may be a dry microbial cell form of the microalgae, but not limited thereto. In addition, the "lysate" of the dry matter collectively calls the result of lysing the dry matter from which moisture is removed in the microalgae culture product, and for example, it may be dry microbial cell powder, but not limited thereto. The culture product of the *Schizochytrium* sp. microalgae may be prepared according to a culture method known in the art by inoculating the microalgae in a microalgae culture medium, and the dry matter of the culture product and lysate thereof may also be prepared according to a treatment or drying method of microalgae or a culture medium known in the art.

The biomass derived from the *Schizochytrium* sp. microalgae may comprise total amino acids of 46 % by weight or more, 48 % by weight or more, 49 % by weight or more, 50 % by weight or more, or 52 % by weight or more, 46 to 60 % by weight, 46 to 55 % by weight, 46 to 53 % by weight, 48 to 53 % by weight, 50 to 55 % by weight, 52 to 55 % by weight, 52 to 53 % by weight, 52.5 to 55 % by weight or 52.5 to 53 % by weight based on the biomass dry weight, and the upper limit may be 80 % by weight or less, 75 % by weight or less, 70 % by weight or less, or 65 % by weight or less, but not limited thereto.

The biomass derived from the *Schizochytrium* sp. microalgae may comprise crude protein of 60 % by weight or more, 64 % by weight or more, 67 % by weight or more, or 70 % by weight or more, 60 to 85 % by weight, 60 to 80 % by weight, 64 to 80 % by weight, 64 to 78 % by weight, 67 to 80 % by weight, 67 to 78 % by weight, 67 to 75 % by weight, 67 to 70 % by weight, 69 to 80 % by weight, 69 to 78 % by weight, 65 to 70 % by weight or 69 to 70 % by weight, based on the biomass dry weight, and the upper limit may be 85 % by weight or less, 80 % by weight or less, 78 % by weight or less, but not limited thereto.

The biomass derived from the *Schizochytrium* sp. microalgae may comprise crude fat of 5 % by weight or more, 7 % by weight or more, 9 % by weight or more, 10 % by weight or more, 14 % by weight or more, 20 % by weight or more, 1 to 20 % by weight, 4 to 23 % by weight, 9 to 25 % by weight, 9 to 23 % by weight, 5 to 18 % by weight, 10 to 18 % by weight, 10 to 15 % by weight, 12 to 18 % by weight, 12 to 15 % by weight or 13 to 15 % by weight based on the biomass dry weight.

The term used in the present description, "crude protein" may be interchangeably used with "crude-protein", and means a protein obtained by measuring total nitrogen in general analysis of food and feed, and multiplying this by 6.25 (reciprocal of the average nitrogen content of protein, 1/0.16).

The term used in the present description, "crude fat" means a component extracted with ether using a Soxhlet extractor in general analysis of food and feed, and "crude fat content" may be interchangeably used with "crude fat amount" or "total lipid" or "total fatty acid."

The term used in the present description, "docosahexaenoic acid (DHA)" is one of polyunsaturated fatty acids having the chemical formula of $C_{22}H_{32}O_2$, and corresponds to omega-3 fatty acids together with eicosapentaenoic acid (EPA), and the common name is cervonic acid, and may also be written in abbreviation as 22:6 n-3.

The term used in the present description, "eicosapentaenoic acid (EPA)" is one of polyunsaturated fatty acids having the chemical formula of $C_{20}H_{30}O_2$, and corresponds to omega-3 fatty acids together with ALA and DHA, and may also be written in abbreviation as 20:5 n-3.

The biomass derived from the *Schizochytrium* sp. microalgae may comprise docosahexaenoic acid (DHA) of 39 % by weight or more, 40 % by weight or more, 41 % by weight or more, 45 % by weight or more, 49 % by weight or more, 50 % by weight or more, 39 to 60 % by weight, 39 to 55 % by weight, 39 to 50 % by weight, 42 to 55 % by weight, 42 to 50 % by weight, 43 to 55 % by weight, 43 to 50 % by weight, 45 to 50 % by weight, 47 to 50 % by weight or 49 to 50 % by weight based on the total crude fat content.

The biomass derived from the *Schizochytrium* sp. microalgae may comprise eicosapentaenoic acid (EPA) of 0.5 % by weight or more, 1.0 % by weight or more, 1.5 % by weight or more, 2.0 % by weight or more, 2.1 % by weight or more, 3.5 % by weight or more, 0.5 % by weight to 15.0 % by weight, 0.5 % by weight to 10.0 % by weight, 0.5 % by weight to 5.0 % by weight, 0.5 % by weight to 3.0 % by weight, 1.0 % by weight to 3.0 % by weight, 1.9 % by weight to 3.0 % by weight, 1.9 % by weight to 2.5 % by weight or 1.9 % by weight to 2.2 % by weight based on the total crude fat content.

The biomass derived from the *Schizochytrium* sp. microalgae may comprise omega-3 fatty acid of 41 % by weight or more, 42 % by weight or more, 50 % by weight or more, 42 to 55 % by weight, 42 to 52 % by weight, 43 to 55 % by weight, 43 to 52 % by weight, 49 to 55 % by weight or 49 to 52 % by weight based on the total crude fat content.

The biomass derived from the *Schizochytrium* sp. microalgae may have pepsin digestibility of 68.7% or more, 69% or more, 70% or more, 73% or more, 75% or more, 77% or more, 80% or more, 83% or more, 89% or more, 69% to 95%, 69% to 90%, 70% to 95%, 70% to 90%, 73% to 95%, or 73% to 90%, 75% to 95%, 75% to 90%, 85% to 95%, 85% to 90%, 87% to 95%, 87% to 90%, 89% to 95% or 89% to 90%.

The term used in the present description, "pepsin digestibility" is used for quality analysis of an animal protein raw material, and means an indicator that can compare how much digestion has been done in a certain environment using pepsin extracted from animal stomach.

The biomass may be cultured by the culture method of *Schizochytrium* sp. microalgae according to one aspect.

Another aspect of the present application provides a composition comprising biomass derived from the *Schizochytrium* sp. microalgae having excellent pepsin digestibility comprising total amino acids of 46 % by weight or more based on the biomass dry weight.

The composition may be characterized by excellent pepsin digestibility, and may be used for food and/or feed preparation.

Other aspect of the present application provides a feed composition comprising biomass derived from the *Schizochytrium* sp. microalgae having excellent pepsin digestibility comprising total amino acids of 46 % by weight or more based on the biomass dry weight.

The biomass derived from the *Schizochytrium* sp. microalgae is as described above.

The term used in the present description, "feed composition" refers to feed fed to animals. The feed composition refers to a material that supplies organic or inorganic nutritional components necessary for maintaining lives of animals or producing meat, milk, and the like. The feed composition may further comprise nutritional components for maintaining lives of animals or producing meat, milk, and the like. The feed composition may be prepared as various forms of feed known in the art, and specifically, concentrated feed, supplementary feed and/or special feed may be included.

The term used in the present description, "feed additive" includes a material added to feed on a purpose of various effects such as nutrient supplement and prevention of body weight loss, enhancement of digestion availability of fibers in feed, oil quality improvement, prevention of reproduction disorder and fertility improvement, summer high-temperature stress prevention, and the like. The feed additive of the present application corresponds to supplementary feed under Control of Livestock and Fish Feed, and may further comprise a mineral matter preparation such as sodium bicarbonate, bentonite, magnesium oxide, complex minerals, and the like, a mineral preparation which is a trace mineral matter such as zinc, copper, cobalt, selenium and the like, a vitamin preparation such as carotene, vitamin E, vitamins A, D, E, nicotinic acid, vitamin B complexes and the like, a protected amino acid preparation such as methionine, lysine and the like, a protected fatty acid preparation such as fatty acid calcium salts, and the like, a living cell such as probiotics (lactic acid bacteria preparations), yeast culture, mold fermented products, and the like, a yeast preparation and the like.

Other aspect of the present application provides a food composition comprising biomass derived from the *Schizochytrium* sp. microalgae having excellent pepsin digestibility comprising total amino acids of 46 % by weight or more compared to the biomass dry weight.

The biomass derived from the *Schizochytrium* sp. microalgae is as described above.

The term used in the present description, "food composition" includes all forms such as functional food, nutritional supplement, health food and food additives and the like, and the food composition of the type may be prepared in various forms according to a common method known in the art.

The composition of the present application may further comprise a dry component consisting of grains, for example, ground or crushed wheat, oats, barley, corn and rice; vegetable protein feed, for example, feed using beans and sunflower as a major component; animal protein feed, for example, blood meal, meat meal, bone meal and fish meal; sugar and dairy products, for example, various kinds of powdered milk and whey powder, and the like, and in addition, it may further comprise a nutritional supplement, a digestion and absorption enhancer, a growth promoter, and the like.

The composition of the present application may be administered to animals alone or in combination with other feed additives in an edible carrier. In addition, the composition may be easily administered to animals as top dressing or directly mixed with feed or as an oral formulation separate from feed. When the composition is administered with feed separately, it may be prepared as an immediate release or sustained release formulation, in combination with a pharmaceutically acceptable edible carrier as well known in the art. Such edible carrier can be solid or liquid, for example, corn starch, lactose, sucrose, soybean flake, peanut oil, olive oil, sesame oil and propylene glycol. When a solid carrier is used, the composition may be top dressing in a tablet, capsule, powder, troche or sugar-containing tablet or microdispersible form. When a liquid carrier is used, the composition may be a formulation of a gelatin soft capsule, or syrup or suspension, emulsion or solution.

The composition of the present application may comprise, for example, a preservative, a stabilizer, a wetting agent or an emulsifier, a cryoprotectant, or an excipient, or the like. The cryoprotectant, may be one or more selected from the group consisting of glycerol, trehalose, maltodextrin, nonfat dry milk and starch.

The preservative, stabilizer or excipient may be comprised in the composition in an effective amount sufficient to reduce deterioration of *Schizochytrium* sp. microalgae comprised in the composition. In addition, the cryoprotectant may be comprised in the composition in an effective amount sufficient to reduce deterioration of *Schizochytrium* sp. microalgae comprised in the composition when the composition is in a dried state.

The composition may be used as being added to feed of animals by immersing, spraying or mixing.

The composition of the present application can be applied to various animal diets, including mammals, birds, fish, crustaceans, cephalopods, reptiles and amphibians, but not limited thereto. For example, the mammal may include pigs, cows, sheep, goats, laboratory rodents or pets or the like, and the bird may include poultry, and the poultry may comprise chickens, turkeys, ducks, geese, pheasant or quail, and the like, but not limited thereto. In addition, the fish may include commercial livestock fish and its fry, ornamental fish and the like, and the crustacean may include shrimp, barnacles and the like, but not limited thereto. Furthermore, the composition may be applied to a diet of a rotifer, which is a zooplankton.

Other aspect of the present application provides a culture method of *Schizochytrium* sp. microalgae, comprising; culturing *Schizochytrium* sp. microalgae under a condition in which a concentration of a residual carbon source in a culture solution is maintained at 6% or less based on the total culture solution.

The *Schizochytrium* sp. microalgae may be *Schizochytrium* sp. microalgae CD01-5004 strain deposited with accession number KCTC14345BP, but not limited thereto.

The biomass derived from the *Schizochytrium* sp. microalgae is as described above.

The term used in the present description, "culturing" means growing the microalgae under an appropriately controlled environmental condition. The culturing process of the present application may be performed according to an appropriate medium and a culture condition known in the art. Such culturing process may be easily adjusted and used by those skilled in the art according to microalgae to be selected.

The term used in the present description, "culture solution" may mean a medium for culturing microalgae, and may mean that a medium for culturing microalgae further comprises microalgae and/or a product produced by microalgae.

The culture condition in the step of culturing *Schizochytrium* sp. microalgae may be specifically performed under a condition in which the concentration of the residual carbon source in the culture solution is maintained at 6% or less, 5% or less, 4% or less, 3% or less, or 2% or less based on the total culture solution.

Specifically, culturing the *Schizochytrium* sp. microalgae of the present application may be performed under a heterotrophic condition, but not limited thereto.

The term used in the present description, "heterotrophic" is a nutritional method which depends on an organic matter obtained from outside the body as an energy source or nutrient source, and is a term corresponding to autotrophic, and may be interchangeably used with the term 'dark culture.'

The culturing *Schizochytrium* sp. microalgae, is not particularly limited thereto, but may be performed by a known batch culture method, continuous culture method, fed-batch culture method or the like. The medium used in the culturing of the microalgae of the present application and other culture conditions may use any one as long as it is a medium used for common culturing of microalgae without particular limitation. Specifically, the microalgae of the present application may be cultured while adjusting temperature, pH and the like under an aerobic condition in a common medium containing suitable carbon sources, nitrogen sources, phosphorus sources, inorganic compounds, amino acids and/or vitamins and the like.

Specifically, appropriate pH (for example, pH 5 to 9, specifically, pH 5 to 8) may be adjusted using a basic compound (e.g.: sodium hydroxide, potassium hydroxide or ammonia) or an acidic compound (e.g.: phosphoric acid or sulfuric acid), but not limited thereto.

The medium used in the culturing *Schizochytrium* sp. microalgae of the present application may be MJW01 medium, but not limited thereto.

The carbon source comprised in the medium used in the culturing *Schizochytrium* sp. microalgae of the present application may be at least one selected from the group consisting of glucose, fructose, maltose, galactose, mannose, sucrose, arabinose, xylose and glycerol, and specifically, it may be at least one selected from the group consisting of glucose, fructose and sucrose, but not limited thereto.

The nitrogen source comprised in the medium used in the culturing *Schizochytrium* sp. microalgae of the present application may be at least one selected from the group consisting of urea, ammonia water, ammonium sulfate $((NH_4)_2SO_4)$, yeast extract, peptone, sodium nitrate $(NaNO_3)$, but it is not limited thereto as long as it is a nitrogen source used for culturing microalgae.

In the medium used in the culturing *Schizochytrium* sp. microalgae of the present application, as a phosphorus source, potassium dihydrogen phosphate, dipotassium hydrogen phosphate, a sodium-containing salt corresponding thereto, and the like may be comprised individually or comprised in combination, but not limited thereto.

The medium used in the step of culturing the *Schizochytrium* sp. microalgae may comprise yeast extract 0.01 to 5 g/L, $KH_2PO_4$ 1.0 to 3.0 g/L, and $K_2HPO_4$ 5 to 20 g/L, but not limited thereto.

In addition, the culturing temperature may be maintained at 20 to 45°C or 25 to 40°C, and it may be cultured for about 10 to 70 hours, but not limited thereto.

The method for culturing the *Schizochytrium* sp. microalgae can increase pepsin digestibility by increasing the total amino acid content based on the microalgae biomass dry weight by supplying the concentration of residual carbon sources in the culture medium to be maintained within a range of 6% or less at maximum based on the total culture medium.

[ADVANTAGEOUS EFFECTS]

The present invention has developed an alternative protein source that can replace fishmeal through a microalgae-based fermentation process. The composition proposed in the present invention not only has a high protein content, but also contains omega-3 fatty acids which are useful fatty acids similar to fishmeal, so that it can directly replace fishmeal when formulating feed. Furthermore, the high-protein microalgae biomass presented in the present invention shows high pepsin digestibility of 70% or more, as a result of pepsin digestibility analysis, so there are advantages in that even if the mixing ratio in feed increases, it does not negatively affect the overall digestibility, and there is no problem of environmental pollution as it is a protein source produced based on microalgae, and it can be stably produced by minimizing influence of external environment.

[BRIEF DESCRIPTION OF THE DRAWINGS]

FIG. 1 is a diagram showing the correlation coefficient between the pepsin digestibility of the microalgae dry biomass and crude fat content in the biomass for each culture condition (mean $\pm$ standard deviation, n=4).
FIG. 2 is a diagram showing the correlation coefficient between the pepsin digestibility of the microalgae dry biomass and crude protein content in the biomass for each culture condition (mean $\pm$ standard deviation, n=4).
FIG. 3 is a diagram showing the correlation coefficient between the pepsin digestibility of the microalgae dry biomass and omega-3 fatty acid content compared to the crude fat weight in the biomass for each culture condition (mean $\pm$ standard deviation, n=4).
FIG. 4 is a diagram showing the correlation coefficient between the pepsin digestibility of the microalgae dry biomass and total amino acid content in the biomass for each culture condition (mean $\pm$ standard deviation, n=4).

[MODE FOR INVENTION]

Hereinafter, the present invention will be described in more detail by examples. These examples are intended only to illustrate the present invention more specifically, and it will be obvious to those skilled in the art that the scope of the present invention is not limited by these examples according to the gist of the present invention.

**<Seed culture of microalgal strain>**

*Schizochytrium* sp. CD01-5004 strain deposited with accession number KCTC14345BP belonging to *Thraustochytrid*-based microalgae was used. The strain was cultured in a 250 mL flask comprising sterilized MJW01 medium (Glucose 30 g/L, $MgSO_4 \cdot 7H_2O$ 3.0 g/L, $Na_2SO_4$ 10 g/L, NaCl 1.0 g/L, yeast extract 9.0 g/L, $MSG \cdot 1H_2O$ 1.0 g/L, $NaNO_3$ 1.0 g/L, $KH_2PO_4$ 0.1 g/L, $K_2HPO_4$ 0.5 g/L, $CaCl_2$ 0.5 g/L, vitamin mixed solution 10 ml/L) under the condition of 26°C, 200 rpm for 30 hours.

**Comparative example 1. Preparation of biomass by culturing strain under condition of 7~9% (w/v ratio) of carbon source concentration compared to culture solution**

The seed cultured strain was aliquoted in a 5L fermenter including sterilized MJW01 medium (Glucose 30 g/L, $MgSO_4 \cdot 7H_2O$ 3.0 g/L, $Na_2SO_4$ 10 g/L, NaCl 1.0 g/L, yeast extract 0.5 g/L, $MSG \cdot 1H_2O$ 1.0 g/L, $NaNO_3$ 5.0 g/L, $KH_2PO_4$ 1.5 g/L, $K_2HPO_4$ 10 g/L, $CaCl_2$ 0.5 g/L, vitamin mixed solution 10 ml/L), thereby conducting culturing under the condition of 28°C, 300 rpm, 0.6 vvm, pH 6.8. During conducting culturing (45 hours), it was supplied so that the concentration of the residual carbon source in the culture solution was maintained within a range of 7~9% (w/v ratio) compared to the total culture solution. Culturing was continued while supplying a nitrogen source from immediately after initiation of the culturing until the end of the culturing. In order to maintain the appropriate nitrogen source concentration, the supply of the nitrogen source was supplied to the culture solution by repeating on/off. As the carbon source, glucose was used, and as the nitrogen source, ammonium sulfate was used. After completing the culturing, by freeze-drying or drum drying of a microbial cell culture solution, dry biomass of Comparative example 1 as a control group was obtained.

**Example 1. Preparation of biomass by culturing strain under condition of 0~2% (w/v ratio) of carbon source concentration compared to culture solution**

During culturing was continued (45 hours) while maintaining the composition of the culture condition (control condition, control group) MJW01 medium of Comparative example 1, it was supplied so that the residual carbon source concentration in the culture solution was maintained within a range of 0~2% (w/v ratio) compared to the total culture solution. The rest culture conditions were culturing by the same method as Comparative example 1 to obtain dry biomass of Example 1.

**Example 2. Preparation of biomass by culturing strain under condition of 2~5% (w/v ratio) of carbon source condition at maximum compared to culture solution**

During culturing was continued (45 hours) while maintaining the composition of the culture condition (control condition, control group) MJW01 medium of Comparative example 1, it was supplied so that the residual carbon source concentration in the culture solution was maintained within a range of 2~5% (w/v ratio) compared to the total culture solution. The rest culture conditions were culturing by the same method as Comparative example 1 to obtain dry biomass of Example 3.

**Example 3. Preparation of biomass by culturing strain under condition of 4~7% (w/v ratio) of carbon source condition at maximum compared to culture solution**

During culturing was continued (45 hours) while maintaining the composition of the culture condition (control condition, control group) MJW01 medium of Comparative example 1, it was supplied so that the residual carbon source concentration in the culture solution was maintained within a range of 4~7% (w/v ratio) compared to the total culture solution.

**Experimental example 1. Component analysis of microalgae dry biomass by culture condition**

**1-1. Analysis of crude fat content and fatty acid content of microalgae dry biomass**

In order to analyze the crude fat content and fatty acid content of each dry biomass obtained in Comparative example 1, and Example 1 to Example 3 above, the following experiment was performed.
Specifically, hydrolysis reaction was performed at 80°C by adding 8.3M hydrochloric acid solution to dry cells of each microalgae 2g of Comparative example 1, and Example 1 to Example 3. Then, after adding ethyl ether 30 mL and petroleum ether 20 mL, a process of mixing for 30 seconds and centrifuging was repeated 3 times or more. The separated solvent layer was transferred to a round flask of which weight was measured in advance, and then it was put into a container from which the solvent and residual moisture were removed through nitrogen purging and dried. By measuring the weight of the oil remained after drying the solvent, the total oil content was calculated, and the omega-3 (DHA and EPA) content comprised in the oil was measured by gas chromatography after progressing pre-treatment with methanolic 0.5N NaOH and 14% trifluoroborane methanol ($BF_3$)

**1-2. Analysis of crude protein content of microalgae dry biomass**

In order to analyze the crude protein content of each dry biomass obtained in Comparative example 1, and Example 1 to Example 3 above, utilizing Kjeldahl automatic analyzer, the following experiment was performed.
Specifically, dry biomass of each microalgae sample obtained in Comparative example 1, and Example 1 to Example 3 was placed in a tube for analysis of Kjeldahl device (Kjeldahl system, Kjeltec 2100), and after degrading in a decomposer by adding a sulfur phase and a catalyst, a decomposition tube was mounted on an auto sampler. By the method of cooling and collecting gas ammonia generated by heat distillation with caustic soda and steam in the Kjeldahl device and then titrating it with hydrochloric acid to automatically calculate the nitrogen content, the crude protein content of the microalgae dry biomass was measured.

**1-3. Analysis of amino acid content of microalgae dry biomass**

In order to analyze the amino acid content of each dry biomass obtained in Comparative example 1, and Example 1 to Example 3 above, the following experiment was performed.
Specifically, after acid hydrolysis of each microalgae dried microbial cell 0.5-1 g of Comparative example 1, and Example 1 to Example 3, total amino acid analysis was performed using liquid chromatography. The analysis result of the individual amino acid was standardized with the amount of the used microalgae dried microbial cells to calculate the content ratio of the individual amino acid in the dried microbial cell, and by adding the content ratios of all amino acids detected as a result of the analysis, the total amino acid content ratio in the microbial cell was calculated.

**1-4. Result of analysis of components of microalgae dry biomass by culture condition**

The result of analyzing the components of each dry biomass obtained in Comparative example 1, and Example 1 to Example 3 by the methods of Experimental example 1-1 to Experimental example 1-3 was shown in Table 1.

[Table 1]

|  | Crude protein /biomass | Total amino acid/bioma ss | Crude fat /biomass | DHA /crude fat | EPA /crude fat | Omega-3 fatty acid /crude fat |
|---|---|---|---|---|---|---|
| Compara tive example 1 | 70.50% | 45.75% | 8.50% | 46.25% | 1.77% | 48.02% |
| Example 1 | 64.10% | **48.10%** | **22.80%** | 40.51% | 1.86% | 42.37% |
| Example 2 | 69.90% | **52.78%** | **14.40%** | **49.40%** | **2.11%** | **51.51%** |
| Example 3 | **77.20%** | **52.18%** | **12.20%** | 42.47% | 1.06% | 43.53% |

As a result, as shown in Table 1, when comparing Comparative example 1 and Example 1, it was confirmed that Example 1 could increase the total amino acid content in the microalgae biomass, but the crude fat content was increased, by maintaining the concentration of the residual carbon source in the culture solution within a low range of 0~2% during the culturing process.

In Example 2, during processing the culturing, the concentration of the carbon source remained in the culture solution was adjusted by 2~5%. When comparing the component contents of Comparative example 1 and Example 2, it was confirmed that in case of maintaining the concentration of the carbon source was maintained within a range of 2~5% in Example 2, the crude protein content was maintained at a level of 70%, and the crude fat content was shown in a middle range of Comparative example 1 and Example 1. In particular, it was shown that the total amino acid content in the biomass showed the highest value among the culture conditions as 52.78%.

In Example 3, during progressing the culturing, the concentration of the carbon source remained in the culture solution was maintained within a range of 4~7%. It was confirmed that when the concentration of the carbon source was maintained within a range of 4~7% in Example 3, the crude protein content was shown as 77.20%, and the total amino acid content was shown as 52.18%, and the crude fat content was shown as 12.20%, so it showed the highest crude protein content among the culture conditions.

**Experimental example 2. Analysis of pepsin digestibility of microalgae dry biomass by culture condition**

Since the digestibility of protein is an important indicator for using microalgae biomass as feed and food, analysis was conducted according to the pepsin digestibility analysis method. In order to analyze the pepsin digestibility of microalgae dry biomass by culture condition, the pepsin digestibility of each dry biomass obtained in Comparative example 1, and Example 1 to Example 3 above was measured by the method disclosed in Association of Official Analytical Chemists, 1990, Official Method of Analysis 971.09 to derive the pepsin digestibility using Equation 1 below.

Specifically, 150 mL of 0.2% pepsin hydrochloric acid solution previously heated to 42 to 45°C was added to 1 g of each dry biomass sample, and it was digested while shaking in a 45°C constant temperature water bath for 16 hours, and then filtered with No. 2A filter paper. After washing fluoride with warm water, the crude protein content of the fluoride and filter paper was obtained by Kjeldahl analysis, and the result was shown in Table 2 below.

The Kjeldahl nitrogen content of Equation 1 was calculated by conducting the method disclosed in the analysis of crude protein utilizing Kjeldahl automatic analyzer of Experimental example 1-3.

[Equation 1]

$$\text{Pepsin digestibility (\%)} = 100 * (A\text{-}B)/A$$

*A: % content of Kjeldahl nitrogen of original sample (Kjeldahl nitrogen in the original sample)
*B: % content of Kjeldahl nitrogen insoluble in acid pepsin solution (Kjeldahl nitrogen insoluble in acid pepsin solution)

[Table 2]

|  | Pepsin digestibility |
| --- | --- |
| Comparative example 1 | 68.59% |
| Example 1 | 73.40% |
| Example 2 | 89.62% |
| Example 3 | 83.2% |

As a result, as shown in Table 2, it was shown that the pepsin digestibility of microalgae dry biomass of Comparative example 1 was relatively low as 68.6%. However, as a result of modifying the biomass component ratio by changing the culture condition, it was confirmed that high pepsin digestibility was shown as the pepsin digestibility of the microalgae dry biomass of Example 1 was 73.40%, and the pepsin digestibility of the microalgae dry biomass of Example 2 was 89.62%, and the pepsin digestibility of the microalgae dry biomass of Example 3 was 83.20%.

**Experimental example 3. Confirmation of correlation between pepsin digestibility and total amino acid content of microalgae dry biomass by culture condition**

As a result of modifying the biomass component ratio through the change of the culture condition in Experimental example 2 above, it was confirmed that the pepsin digestibility was increased, and thus, it was confirmed which indicator among the components of the biomass had a correlation with the pepsin digestibility.

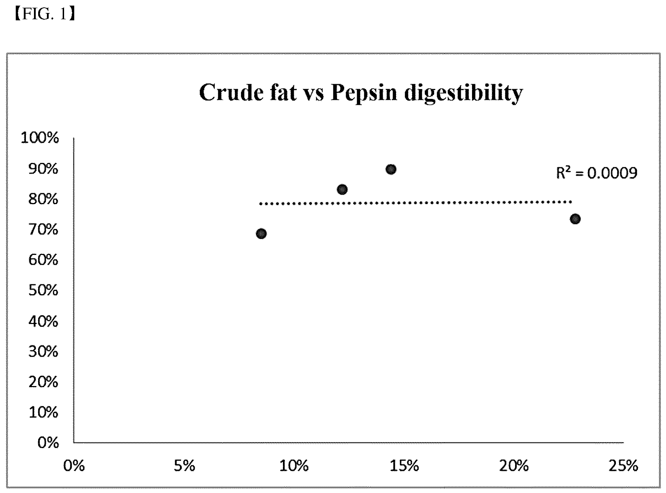

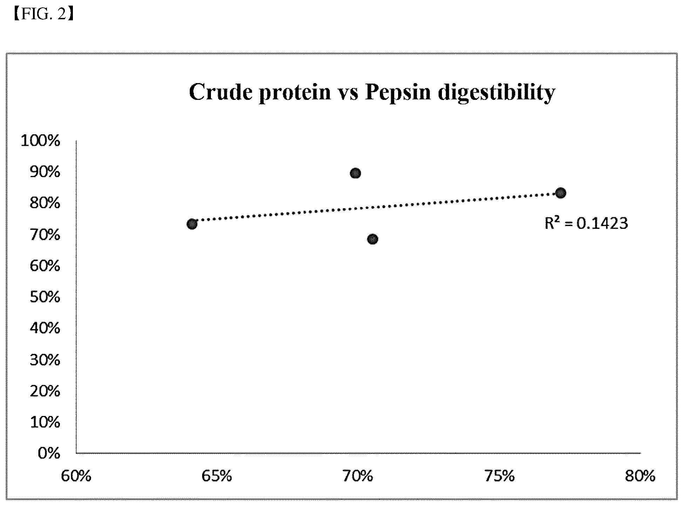

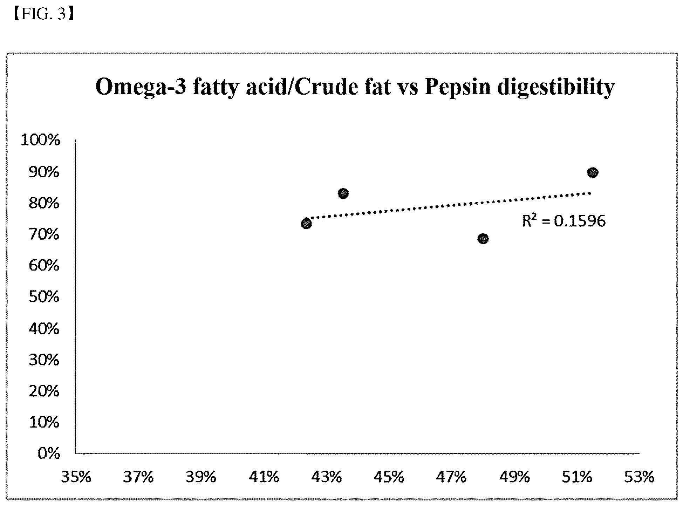

Specifically, correlation coefficients between the crude fat content, crude protein content, omega-3 fatty acid/crude content and total amino acid content and pepsin digestibility of each dry biomass obtained in Comparative example 1, and Example 1 to Example 3 were derived, and the result was shown in FIG. 1 to FIG. 4.

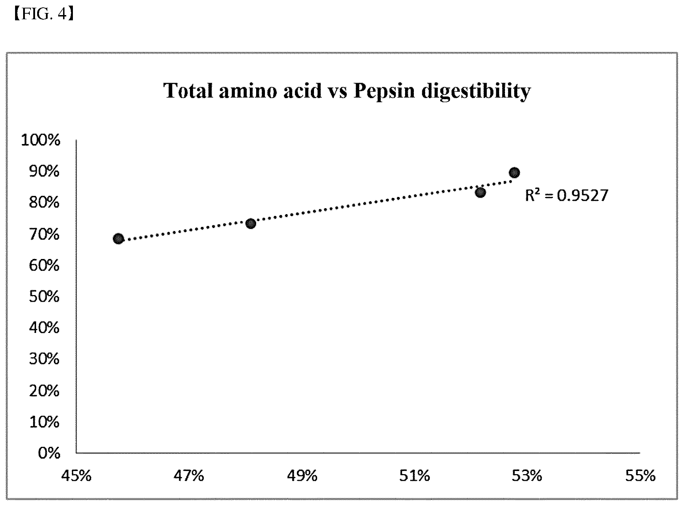

As a result, as shown in FIG. 1 to FIG. 3, it was shown that the omega-3 fatty acid content compared to the crude fat content, crude protein and crude fat weights among the biomass components had no significant correlation. On the other hand, as shown in FIG. 4, it was shown that there was significant correlation between the total amino acid content in the biomass and pepsin digestibility ($R^2$=0.9527). Since total amino acids is a concept which is included in the crude protein content, the crude protein content and total amino acids are known as values that fluctuate in proportion, but it can be confirmed that as described in the present invention, even if the crude protein content is similar, the total amino acid content can be changed depending on the fermentation condition, and the pepsin digestibility increases as the total amino acid content in the biomass increases.

[Accession number]

Name of depository authority: Korea Research Institute of Bioscience and Biotechnology, Korean Collection for Type Cultures (KCTC) Accession number: KCTC14345BP Date of deposit: 20201026

# EP 4 512 888 A1

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: CJ CHEILJEDANG CORPORATION

CJ CHEILJEDANG CORPORATION

330, Dongho-ro, Jung-gu, Seoul

Republic of Korea

| I. IDENTIFICATION OF THE MICROORGANISM | |
|---|---|
| Identification reference given by the DEPOSITOR: <br><br> *Schizochytrium* sp. **CD01-5004** | Accession number given by the INTERNATIONAL DEPOSITARY AUTHORITY: <br><br> **KCTC 14345BP** |

| II. SCIENTIFIC DESCRIPTION AND/OR PROPOSED TAXONOMIC DESIGNATION |
|---|
| The microorganism identified under I above was accompanied by: <br><br> [ ] a scientific description <br><br> [ ] a proposed taxonomic designation <br><br> (Mark with a cross where applicable) |

| III. RECEIPT AND ACCEPTANCE |
|---|
| This International Depositary Authority accepts the microorganism identified under I above, which was received by it on **October 26, 2020.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures** <br><br> Address: Korea Research Institute of Bioscience and Biotechnology (KRIBB) 181, Ipsin-gil, Jeongeup-si, Jeolllabuk-do 56212 Republic of Korea | Signature(s) of person(s) having the power to represent the International Depositary Authority or of authorized official(s): <br><br> *Song-Gun Kim* <br><br> KIM, Song-Gun, Director <br> Date: **October 26, 2020** |

Form BP/4 (KCTC Form 17)                                                                 sole page

**Claims**

1. A preparation method of biomass derived from the *Schizochytrium* sp. microalgae, comprising: culturing *Schizochytrium* sp. microalgae under a condition in which a concentration of a residual carbon source in a culture solution is maintained at 7% or less based on the total culture solution.

2. The preparation method of biomass derived from the *Schizochytrium* sp. microalgae according to claim 1, wherein the *Schizochytrium* sp. microalgae is *Schizochytrium* sp. CD01-5004 strain deposited with accession number KCTC14345BP.

3. The preparation method of biomass derived from the *Schizochytrium* sp. microalgae according to claim 1, wherein the culturing is performed in MJW01 medium.

4. The preparation method of biomass derived from the *Schizochytrium* sp. microalgae according to claim 1, wherein the carbon source of the medium is at least one selected from the group consisting of glucose, fructose and sucrose.

5. The preparation method of biomass derived from the *Schizochytrium* sp. microalgae according to claim 1, wherein the medium comprises at least one selected from the group consisting of urea, ammonia water, ammonium sulfate, yeast extract, peptone and sodium nitrate as a nitrogen source.

6. The preparation method of biomass derived from the *Schizochytrium* sp. microalgae according to claim 1, wherein the medium comprises yeast extract 0.01 to 5 g/L, $KH_2PO_4$ 1.0 to 3.0 g/L, and $K_2HPO_4$ 5 to 20 g/L.

7. The preparation method of biomass derived from the *Schizochytrium* sp. microalgae according to claim 1, wherein the culturing is performed for 10 to 70 hours.

8. The preparation method of biomass derived from the *Schizochytrium* sp. microalgae according to claim 1, wherein the microalgae culturing method increases the pepsin digestibility by increasing the total amino acid content based on the microalgae dry weight.

9. The preparation method of biomass derived from the *Schizochytrium* sp. microalgae according to claim 1, wherein the pepsin digestibility is 70% or more.

10. A biomass derived from the *Schizochytrium* sp. microalgae having excellent pepsin digestibility prepared by the preparation method of claim 1.

11. The biomass according to claim 10, wherein the *Schizochytrium* sp. microalgae is *Schizochytrium* sp. CD01-5004 strain deposited with accession number KCTC14345BP.

12. The biomass according to claim 10, wherein the biomass contains 60 % by weight or more of crude protein based on the biomass dry weight.

13. The biomass according to claim 10, wherein the biomass contains 4 % by weight to 23 % by weight of crude fat based on the biomass dry weight.

14. The biomass according to claim 10, wherein the biomass contains 40 % by weight or more of docosahexaenoic acid based on the total crude fat weight.

15. The biomass according to claim 10, wherein the biomass contains 0.5 % by weight or more of eicosapentaenoic acid based on the total crude fat weight.

16. The biomass according to claim 10, wherein the biomass contains 41 % by weight or more of omega-3 fatty acid based on the total crude fat weight.

17. The biomass according to claim 10, wherein the biomass has 70% or more of pepsin digestibility.

18. A feed composition comprising the biomass derived from the *Schizochytrium* sp. microalgae of claim 10.

**19.** A food composition comprising the biomass derived from the *Schizochytrium* sp. microalgae of claim 10.

【FIG. 1】

Crude fat vs Pepsin digestibility

$R^2 = 0.0009$

【FIG. 2】

**Crude protein vs Pepsin digestibility**

$R^2 = 0.1423$

【FIG. 3】

**Omega-3 fatty acid/Crude fat vs Pepsin digestibility**

$R^2 = 0.1596$

【FIG. 4】

**Total amino acid vs Pepsin digestibility**

$R^2 = 0.9527$

## INTERNATIONAL SEARCH REPORT

| International application No. |
|---|
| **PCT/KR2023/001183** |

### A. CLASSIFICATION OF SUBJECT MATTER

**C12N 1/12**(2006.01)i; **C12P 1/00**(2006.01)i; C12R 1/89(2006.01)n

According to International Patent Classification (IPC) or to both national classification and IPC

### B. FIELDS SEARCHED

Minimum documentation searched (classification system followed by classification symbols)

C12N 1/12(2006.01); A23K 10/16(2016.01); A23L 33/12(2016.01); A23L 33/195(2016.01); C12N 1/02(2006.01); C12P 23/00(2006.01); C12P 7/64(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 스키조키트리움 속(Schizochytrium sp.), 도코사헥사엔산(docosahexaenoic acid: DHA), 에이코사펜타엔산(eicosapentaenoic acid: EPA), 펩신 소화율(pepsin digestibility), 탄소원(carbon source), 배지 (medium)

### C. DOCUMENTS CONSIDERED TO BE RELEVANT

| Category* | Citation of document, with indication, where appropriate, of the relevant passages | Relevant to claim No. |
|---|---|---|
| X | KR 10-2018-0029249 A (FERMENTALG et al.) 20 March 2018 (2018-03-20)<br>    See claims 1-7 and 11-23; and paragraphs [0009]-[0011]. | 1-19 |
| A | KR 10-2020-0074047 A (PHYCOILBIOTECH KOREA, INC.) 24 June 2020 (2020-06-24)<br>    See claims 1-14. | 1-19 |
| A | US 2016-0298149 A1 (ROQUETTE FRERES) 13 October 2016 (2016-10-13)<br>    See entire document. | 1-19 |
| A | KR 10-1847551 B1 (INDUSTRIAL COOPERATION FOUNDATION JEONBUK NATIONAL UNIVERSITY et al.) 10 April 2018 (2018-04-10)<br>    See entire document. | 1-19 |
| A | KR 10-2011-0037588 A (DAESANG CORPORATION) 13 April 2011 (2011-04-13)<br>    See entire document. | 1-19 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **26 April 2023** | **26 April 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/001183**

| Patent document cited in search report | | | Publication date (day/month/year) | Patent family member(s) | | | Publication date (day/month/year) |
|---|---|---|---|---|---|---|---|
| KR | 10-2018-0029249 | A | 20 March 2018 | AU | 2016-294730 | A1 | 25 January 2018 |
| | | | | BR | 112018000847 | A2 | 04 September 2018 |
| | | | | CA | 2992148 | A1 | 26 January 2017 |
| | | | | CN | 108026503 | A | 11 May 2018 |
| | | | | CO | 2018001420 | A2 | 22 November 2018 |
| | | | | EP | 3325605 | A1 | 30 May 2018 |
| | | | | FR | 3038913 | A1 | 20 January 2017 |
| | | | | FR | 3038913 | B1 | 01 May 2020 |
| | | | | IL | 256928 | A | 29 March 2018 |
| | | | | IL | 256928 | B | 25 March 2021 |
| | | | | JP | 2018-528785 | A | 04 October 2018 |
| | | | | MX | 2018000692 | A | 06 September 2018 |
| | | | | PH | 12018500130 | A1 | 23 July 2018 |
| | | | | RU | 2018103326 | A | 19 August 2019 |
| | | | | SG | 11201800120 | A | 27 February 2018 |
| | | | | US | 2018-0208886 | A1 | 26 July 2018 |
| | | | | WO | 2017-012931 | A1 | 26 January 2017 |
| KR | 10-2020-0074047 | A | 24 June 2020 | None | | | |
| US | 2016-0298149 | A1 | 13 October 2016 | AU | 2014-369524 | A1 | 09 June 2016 |
| | | | | AU | 2014-369524 | B2 | 12 April 2018 |
| | | | | CA | 2931233 | A1 | 25 June 2015 |
| | | | | CA | 2931233 | C | 08 November 2022 |
| | | | | CN | 105829540 | A | 03 August 2016 |
| | | | | CN | 105829540 | B | 22 November 2019 |
| | | | | EP | 3083973 | A1 | 26 October 2016 |
| | | | | EP | 3083973 | B1 | 08 May 2019 |
| | | | | ES | 2738672 | T3 | 24 January 2020 |
| | | | | FR | 3015516 | A1 | 26 June 2015 |
| | | | | FR | 3015516 | B1 | 22 January 2016 |
| | | | | JP | 2017-500044 | A | 05 January 2017 |
| | | | | JP | 6584409 | B2 | 02 October 2019 |
| | | | | KR | 10-2016-0098233 | A | 18 August 2016 |
| | | | | KR | 10-2247276 | B1 | 03 May 2021 |
| | | | | LT | 3083973 | T | 25 September 2019 |
| | | | | NZ | 720322 | A | 27 May 2022 |
| | | | | US | 10392636 | B2 | 27 August 2019 |
| | | | | WO | 2015-092301 | A1 | 25 June 2015 |
| KR | 10-1847551 | B1 | 10 April 2018 | None | | | |
| KR | 10-2011-0037588 | A | 13 April 2011 | KR | 10-1068520 | B1 | 28 September 2011 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220047721
- KR 1564829